# EUROPEAN PATENT APPLICATION

(11) **EP 4 328 308 A1**
(43) Date of publication of application: **28.02.2024**
(21) Application number: 23155034.4
(22) Date of filing: 06.02.2023
(51) Int. Cl.: C12N 15/10, C12N 9/00, C12P 21/02

(54) **MODIFIED TRNA-SYNTHETASE FOR THE INCORPORATION OF NON-CANONICAL AMINO ACIDS**

(30) Priority: 22.08.2022 DE 102022121159
(71) Applicant: Technische Universität Berlin, 10623 Berlin (DE)
(72) Inventor: Koch, Nikolaj, Berlin (DE); Budisa, Prof. Dr. Nediljko, Feldkirchen (DE)
(74) Representative: Fleuchaus & Gallo Partnerschaft mbB

(57) **Abstract**

The present invention refers to modified enzymes, namely mutated tRNA synthetases and provides a system employing such modified enzymes together with tRNA-pairs as well as a platform for the generation of site-specifically modified, recombinant polypeptides or proteins - *iter alia* - for scientific or medical purposes.

## Description

### Field of the Invention

The present invention refers to modified enzymes, namely mutated tRNA synthetases and provides a system employing such modified enzymes together with tRNAs-pairs as well as a platform for the generation of site-specifically modified, recombinant polypeptides or proteins - *iter alia* - for scientific or medical purposes.

### Background

In the field of synthetic biology the expanding of the genetic code has proven to be an important tool for adding new chemistries to the biological world and expand the chemical space of proteins beyond the standard 20 amino acids. In this regard, amino acids with non-proteinogenic functional groups (ncAAs) can be used to manipulate, design, and elucidate protein structure, dynamics, function, allosterism, interactions, catalysis, folding, synthesis, trafficking, degradation, and aggregation.

With such approaches unnatural or artificial proteins are made based on novel sequences which have no prior existence in nature and that have unique new-to-nature functionalities. Previous approaches modified recombinant proteins with functionalities such as e. g. photoisomerizability, new types of spectroscopic markers and elements like boron, fluorine or orthogonal chemical groups, novel steric and folding properties.

Thus, it seems that the engineering of orthogonal aminoacyl-tRNA-synthetase/tRNA-pairs for site-specific incorporation of non-canonical amino acids (ncAAs) has become one tool for synthetic biology to create orthogonal translation systems (OTSs).

In the context of this description an OTS is understood as a system of an orthogonal aminoacyl-tRNA synthetase (aaRS) together with a cognate tRNA pair, within a recombinant protein expression system. The orthogonal tRNA must not interact with the endogenous aaRS and is just recognized from the introduced aaRS, while the same aaRS must not recognize one of the 20 canonical amino acids. The protein production system can be a cellular host (bacteria, yeast, mammalian cells) or cell-free.

As the physical properties of ncAAs can be precisely defined and their incorporation into proteins can be site-specifically encoded for with high fidelity, the hereinafter described method is a developing way to manipulate and design protein functionalities or elucidate their properties.

Several developments have been pre-described for incorporating ncAAs into recombinant proteins e.g. in WO2009056803A1 and WO2011045316A1 and more than 200 ncAAs have been created for incorporation into proteins via various genetic code expansion routes.

Most OTSs presently known are derived from the *Methanosarcina mazei*/*barkeri* pyrrolysyl-tRNA synthetases (*Mm*PylRS/*Mb*PylRS) or the *Methanocaldococcus jannaschii* tyrosyl-tRNA synthetase (*Mj*TyrRS). Unlike all other OTSs, the PylRS system is particularly useful because it can be used in all three domains of life due to its natural orthogonality. Also, PylRS/tRNA^{Pyl} pairs from the species of *Methanosarcina* are a known choice for genetic code expansion due to their orthogonal reactivity in both bacterial and eukaryotic cells.

In WO2015011081A1 methods have been pre-described for incorporating ncAAs into recombinant proteins. One major drawback of these and all known PylRS derived systems to date is the low catalytic efficiency which results in low ncAA incorporation yields.

Many efforts have been made to gradually increase the PylRS-based OTS efficiency for wild-type and engineered systems. These include optimization of OTS plasmid copy numbers and promotor strength of aminoacyl-tRNA synthetases (aaRSs) and/or tRNA genes, engineering of tRNA^{Pyl}, rational and semi-rational engineering of aaRS, optimization of sequence context around the target codon, host cell engineering, engineering of parts of the translational machinery (e.g., elongation factor TU) and increasing solubility of the aaRS.

Nevertheless, up to now the yield of recombinant protein production falls short and sometimes far of that of the wild-type reference protein. With such moderate efficacy any results scientific research was always prone to be accidentally artefacts, only. Thus, to fully exploit all the valuable properties of such PylRS system or OTS in general, the drawbacks of the prior developments need to be overcome.

This problem is solved, by providing a catalytically highly active tRNA-Synthetase, which shows a substantially increased catalytic efficiency due to newly introduced mutations in the catalytic centre of the mutated tRNA-Synthetase (mRS) as of Claim 1, which is further characterized in the dependent claims.

The present invention also provides the method and the teaching to use the improved tRNA-Synthetase together with suitable, optionally also modified tRNAs to be used on modified target sequences aiming to generate new classes of recombinant polypeptides and proteins.

For this the inventors provided a mutated tRNA synthetase (mRS). This newly modified synthetase derives from *Methanococcoides burtonii* (*Mbur*PylRS) and has at least 90% sequence identity with *Mbur*PylRS. It is explicitly characterized by one or more modification in the catalytic centre. The most relevant and essential mutation in the catalytic centre of the newly provided tRNA synthetase is an amino acid exchange Y346F, wherein the position of the amino acids to be mutated or modified is based on the numbering od the amino acid sequence of *Mbur*PylRS, which is accessible in the Genbank under the Accession number: Q12UB6.1

The present invention is based upon the remarkable finding that one mutation in the catalytic centre of a tRNA-synthetase improves the efficiency of the integration or/and incorporation of ncAAs into a newly generated recombinant protein or polypeptide. Due to this surprising finding, that the above-mentioned mutation in the catalytic centre of the mRS has the capacity to increase the catalytic efficiency of the mRS, a new chapter in synthetic biology is to start.

The present invention provides a mutated tRNA synthetase (mRS) deriving from *Methanococcoides burtonii* (*Mbur*PylRS), which is characterized by one specific modification, wherein the amino acid (AA) on position 346 is exchanged from Y towards F.

Further studies have shown that the mutated tRNA synthetase according to the invention can additionally carry one or multiple further amino acid exchanges selected from the group consisting of N308Q, N308L, N308M, N308G, N308A, C310A, C310G, C310W, S379T and W379T. While the inventors tested multiple amino acid modifications, only the modifications as indicated can be correlated with either an increase in efficiency and/or a stabilizing effect, be it stabilising the binding capacity of the tRNA or stabilising the catalytic centre of the mRS.

Additionally, the present invention provides in further embodiments variants of the mutated tRNA synthetase which carry different combinations of amino acid exchanges selected from the above list. Such variants of the mRS comprise e.g. the combination of amino acid exchanges selected from the group of variants with the following amino acid exchanges N308A:C310A:Y346F, N308G:C310G:Y346F, N308M:C310W:Y346F, N308Q:C310W:Y346F, N308L:C310W:Y346F, C310W:Y346F:S379T, and a humanized sequences carrying the amino acid exchanges C310W:Y346F:W379T.

Alternative pairings of one or more of the above identified amino acid exchanges, namely N308Q, N308L, N308M, N308G, N308A, C310A, C310G, C310W, S379T, W379T. are also advantageous to variant degrees and thus, included as further embodiments of the present teaching.

In the context of the present document the term "mutation" or "modification" of the amino acid (AA) sequence are used to express that in an AA sequence an exchange of one or more AA has been introduced, whether such exchange has been introduced by mutating the genetic code and thus the encoding nucleotide sequence or by artificially modifying the protein sequence is interchangeable. In the context of the description an amino acid exchange is described by indicating the original AA then the position in the sequence and then the modified amino acid. For example, N308Q means that the amino acid "N" located at sequence position 308 is exchanged against the amino acid "Q".

The mutated tRNA-Synthetase (mRS) of the invention has a homology to the progenitor tRNA-synthetase of *Methanococcoides burtonii* of at least 90%, preferably at least 93%, 95%, 98% or even 99%. The claimed and mutated tRNA-Synthetase (mRS) is primarily identified and defined by the one amino acid modification - as mentioned above - and in this case shows still a sequence identity of 99% when compared to the parent *Mbur*PylRS.

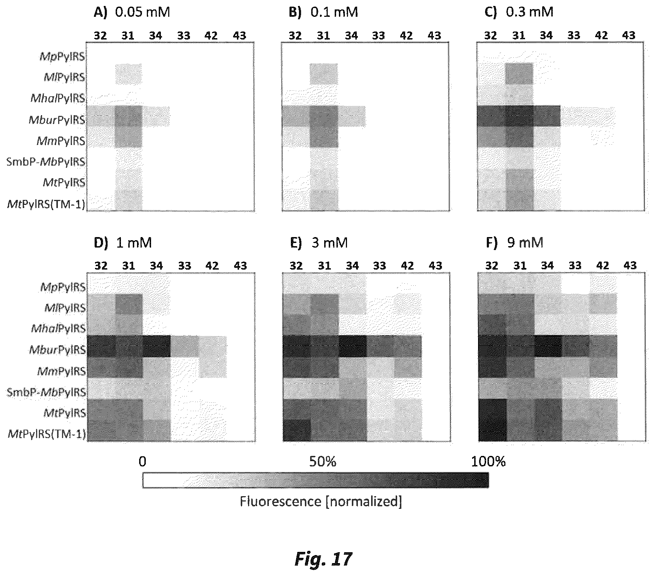

It was shown that the claimed mRS are substantially different in their catalytic efficiency and specificity. Such comparison is particularly founded on efficiency data comparing the newly provided mRS with already known PylRS systems (Figure 17).

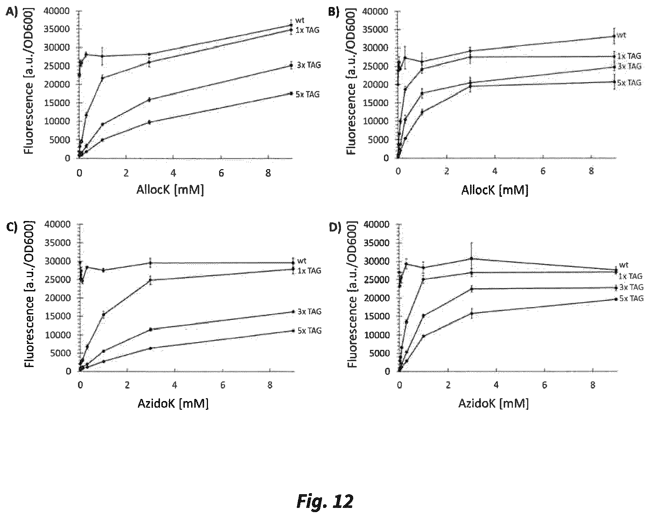

In the Examples it is - *iter alia* - shown that the different mRSs according to the different embodiments of the invention, which carry one or several mutations as mentioned above, show an increased catalytic efficiency when compared to the other known PylRS enzymes and actually show individual results with up to 80% or even 300% improved performance (Figure 12).

Furthermore, the second or more additional mutation of the mRS of the present invention further improve stability of the catalytic centre, binding capacity by the catalytic centre, improve enzymatic activity and efficiency and/or improve target specificity.

The mRS according to the present invention can be further described by the consensus sequence (SEQ ID NO: 1): 
MEKQLLDVLVELNGVWLSRSGLLHGIRNFEITTKHIHIETDCGARFTVRNSRSSRSARSLRHNKYRKPCKRCRPADEQIDR
FVKKTFKEKRQTVSVFSSPKKHVPKKPKVAVIKSFSISTPSPKEASVSNSIPTPSISVVKDEVKVPEVKYTPSQIERLKTLMS
PDDKIPIQDELPEFKVLEKELIQRRRDDLKKMYEEDREDRLGKLERDITEFFVDRGFLEIKSPIMIPFEYIERMGIDKDDHLN
 
KQIFRVDESMCLRPMLAPCLYNYLRKLDKVLPDPIRIFEIGPCYRKESDGSSHLEEFTMVX2FX3QMGSGCTRENMEALIDEF
LEHLGIEYEIEADNCMVX1GDTIDIMHGDLELSSAVVGPIPLDREWGVNKPX4MGAGFGLERLLKVRHNYTNIRRASRSELYY
NGINTNL,
 wherein in (346)X1 is **F** and wherein (308)X₂ is N, Q, L, M, G or A, wherein (310)X₃ is C, G, W or A and wherein (379)X₄ is S or T.

According to further embodiments some particularly usefull mRS are defined by the aminio acid sequences as described in SEQ ID No: 3 to 9.

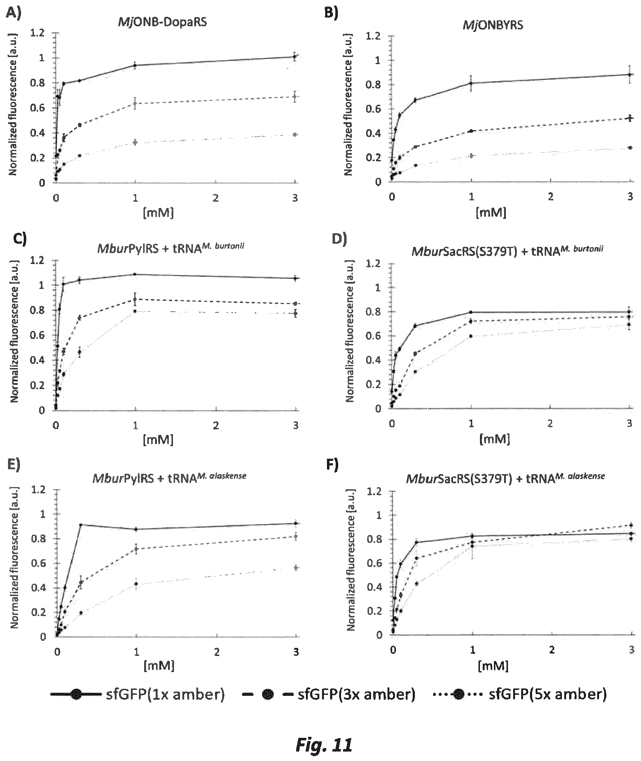

The mRS according to the present invention proved far superior regarding the incorporation of ncAAs at multiple sites, particularly if compared to one of the best performing OTS known, namely *Mj*TyrRS (Figure 11). These results are particularly important since the technological field is presently wishing for moving from single site ncAA incorporation with one ncAA to multi-site incorporation with multiple ncAAs. Consequently, a further need for more catalytically active enzymes has developed.

While a known and prevalent approach to genetic code expansion is to use thermophilic aaRS to generate necessary mutations, in this work the inventors show that the presently claimed mRS is a special case where a modified psychrophilic homolog of *Methanococcoides burtonii* (*M. burtonii*) demonstrated its superiority, not only in catalytic efficiency but also in substrate diversity.

For this the inventors have engineered psychrophilic ("cold") PylRS orthologs to a so called 'cold-OTS' as an alternative to the commonly used meso- and thermophilic OTSs.

In particular, the newly provided mRS is, thus, a psychrophilic homolog of PylRS from *Methanococcoides burtonii* having a high sequence homology with the parent enzyme *Mbur*PylRS but carrying one essential modification as mentioned above and showing remarkable properties in terms of catalytic efficiency and promiscuity. The newly provided mRS is thus part of a new OTS, herein further addressed as cold-OTS.

As already the parent enzyme, PylRS, has a wide range of host organisms, such as archaea, bacteria, and eukaryotes, the newly provided mRS, i.e. the new cold-OTS as described herein will greatly facilitate the transformation of the expanded genetic code in various reigns and with a high-value in chemistry-driven biotechnology.

Additional advantages can be exploited by using humanised sequences of the mRS coding sequence, as e.g. described in SEQ ID 10. Additionally, the mRS and the humanised mRS enzyme can be further optimised by introducing an optionally codon optimised nuclear export sequence (NES), e.g. SEQ ID no: 11, right after the start codon (ATG) at the 5' end at of the various mRS of the present invention.

According to further examples it has been shown that the mutated tRNA synthetases (mRS) according to the present invention are particularlysuitable for the incorporation of multiple amino acids with non-proteinogenicfunctional groups (ncAAs) into newly synthesized, recombinant protein or polypeptides, wherein the incorporation is not only site-specific but was further shown to be statistically increased or also in a stochastic manner a combination of natural amino acids and ncAAs.

In the context of the present document and for a more complete understanding the basic principles of synthetic biology and the structure of the genetic code as well as protein biosynthesis are briefly summarized.

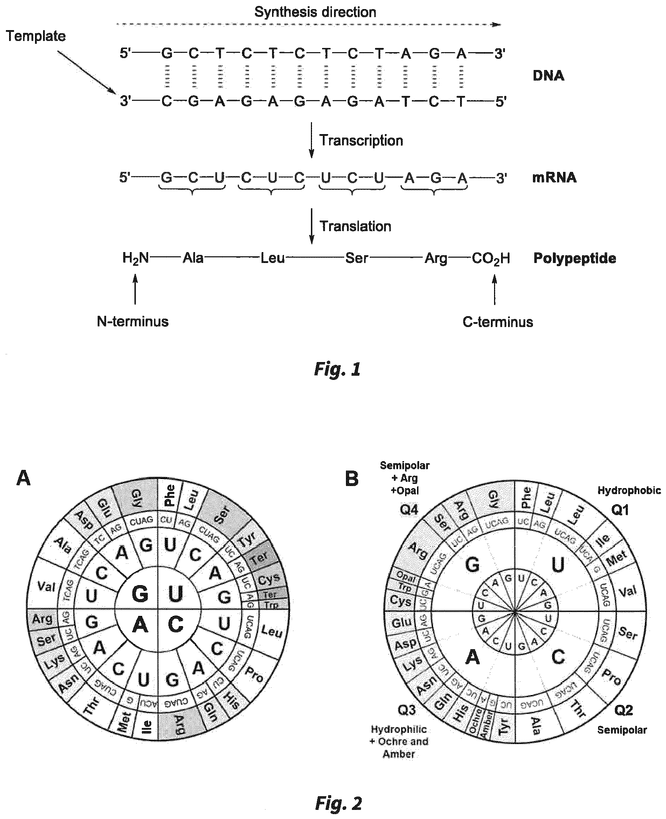

Protein biosynthesis starting from DNA is illustrated in Figure 1. It is well known that genes are the essential part of the genetic information. The genes which encode the amino acid sequence of a protein is specified by DNA nucleotide bases in a defined manner, so called base pair triplets (Figure 2).

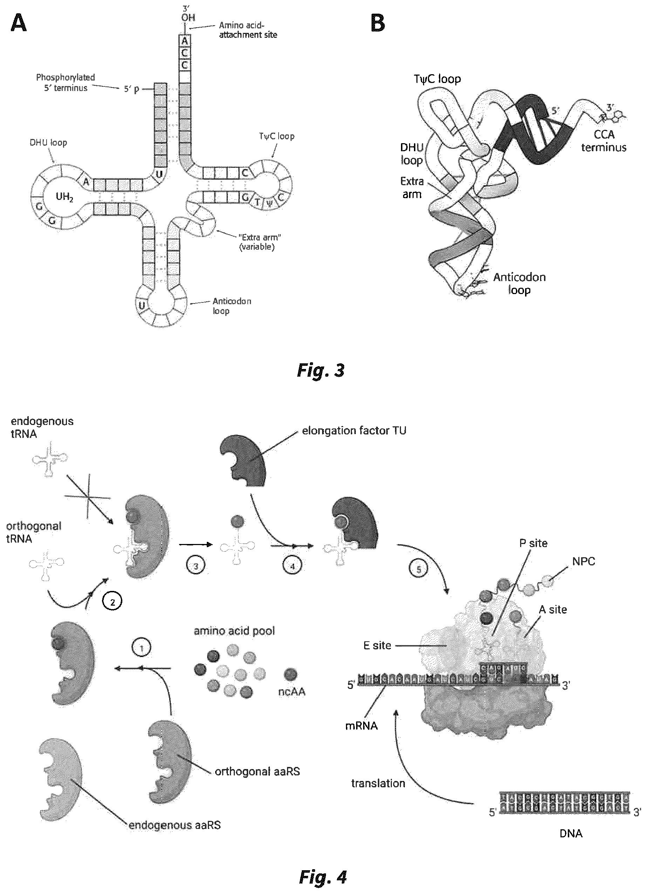

For the translation of mRNA into a correct amino acid sequence, an adapter molecule is used, which is known as transfer RNA (tRNA). All known tRNAs consist of 73 to 93 ribonucleotides. They are composed of several nonstandard chemically altered nucleobases, with methylation as the simplest modification. These modifications modulate recognition interactions with tRNA-synthetase and the ribosome. In canonical two-dimensional representation, tRNAs resemble a cloverleaf (Figure 3). Since there are 61 codons, a tRNA must be able to decode more than one codon. This exact mechanism was postulated by C_{RICK} as the "Wobble Hypothesis" which states, that only the first two bases form a strict Watson-Crick base pairing. Such wobble decoding is often enabled by posttranscriptional modifications of the first anticodon base. One of the most important features of the tRNA is its anticodon. With the codon-anticodon recognition, the tRNA assigns a specific AA to each codon in a mRNA sequence. For protein translation to occur with high fidelity, the tRNA must be loaded with the correct AA. This function is executed by aminoacyl tRNA synthetases (aaRSs), a term and abbreviation that is in the context of the application used to identify unmodified tRNA-synthetases.

The fidelity of aaRS in AA recognition and tRNA loading is of paramount importance for the correct translation of mRNA. A robust decoding mechanism is only possible when this process takes place with a very low error rate. The error rate is estimated to be 1 - 6·10⁻⁴ per codon translated.

Most species possess at least 20 aaRS. Interestingly, *Methanococcoides burtonii,* has one very distinct additional 21st aaRS. It is particularly of interest as this 21^{st} aaRS, the so-called PylRS, which is the starting point for the modified mRS of the present application.

There are some known cases in which an organism possesses fewer than 20 (for which there are various compensatory strategies). Although the catalyzed reaction is always the same, aaRSs differ in size, structure, and subunit composition. After the loaded tRNA is released from the aaRS, the charged tRNA binds to the elongation factor TU (EF-TU) which guides it to the ribosome. At the same time, the ribosome binds to the mRNA and the codon-anticodon recognition mechanism can take place. The ribosome consists of three subunits which is composed of over 50 proteins and ribosomal RNA (rRNA) (Figure 4).

In the area of synthetic biology, the above-described knowledge is systematically used to manipulate a biological system by so called forward engineering.

In this regard it is to acknowledge that the inventors applied two approaches. They not only improved the catalytic efficiency of the tRNA synthetase by providing several improved mRS, but also provided modified tRNAs to establish a so-called `cold OTS' for the generation of recombinant polypeptides and proteins.

Accordingly, the mRS according to the invention are particularly useful as they can integrate ncAAs into a newly synthesized polypeptide or protein by elongating the growing amino acid sequence with tRNAs selected from the group of homologous tRNAs, heterologous tRNAs and engineered tRNAs.

In the context of the description the term "homologue tRNAs" needs to be understood as those tRNAs, which would be naturally occurring in the cellular, bacterial or archaea system, in which the mRS of the invention is put to action.

The term "homologous or homologue tRNAs" refers on the one hand to the tRNAs encoded in the genome of the specific cell or microorganism of interest from which also the corresponding aaRS originates. On the other hand, the term "homologue tRNAs" can be used to describe the tRNAs expressed in a host cell or among others the OTS-system, into which the mRS of the invention is introduced. For example, strain E. *coli* K-12 has 85 to 86 homologous tRNA copies in its genome, when now a mRS according to the invention is introduced into a *E.coli* then the tRNAs of the *E.coli* would be identified as homologous tRNAs, while additional tRNA sequences, which are genetically modified or derive from other organisms, and which are also introduced (e.g. by plasmids) to *E.coli* would be identified as heterologous tRNAs.

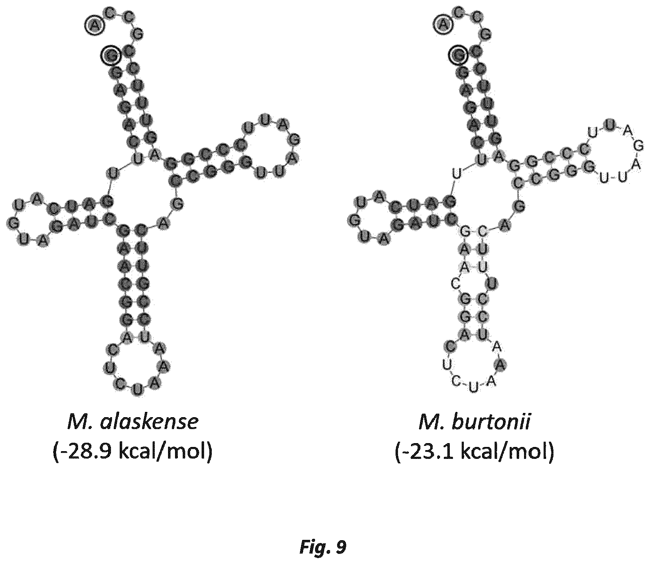

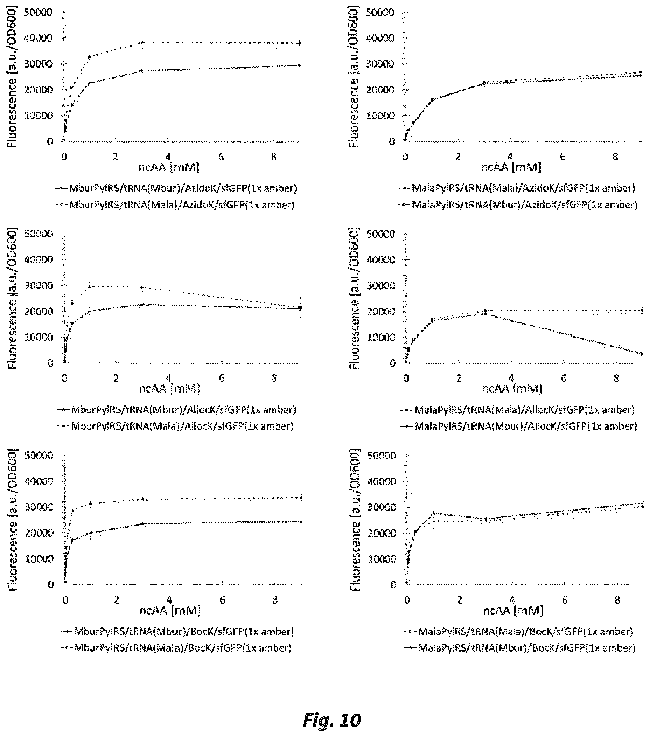

Consequently, the term "heterologous or heterologue tRNAs" also covers tRNA sequences which derive from the genome of an organism different to the organism of which the mRS employed originates. One example for a preferred heterologous tRNA is tRNA^{Pyl} of the organism *Methanococcoides alaskense* (*M. alaskense*)*.* This heterologous tRNA has been tested together the mRS of the invention and it was found that the efficiency of corresponding cold-OTS was further improved for different substrates by up to 50% (Figure 10). Without being bound by the theory, it may be one explanation for such surprising increase that the structure of the *M. alaskense* tRNA^{Pyl} is more stable in the chosen cell culture cultivation conditions of the OTS according to the invention. It was found that at the chosen cell cultivation conditions the *M*. *alaskense* tRNA^{Pyl}showed a predicted decrease in Gibbs free energy of -28,9 kcal/mol, while under the same conditions the *M*. *burtonii* tRNA^{Pyl} showed a Gibbs free energy of -23,1 kcal/mol (Figure 9).

In the field of synthetic biology, it is particularly interesting to use mRS as of the present invention in a heterologous cellular or microbial system, which includes organisms of the reign of bacteria, fungi or archaea, to employ said mRS together with homologous, heterologous and/or engineered tRNAs in the context of the `cold OTS' for the production of new and/or recombinant proteins translated in such expression system.

According to further embodiments the mRS of the invention are particularly useful for integrating ncAAs into a newly synthesized polypeptide or protein by elongating the growing amino acid sequence by recognizing and binding specifically engineered or modified tRNAs.

In the context of the present description the term "engineered or modified tRNAs" covers all RNAs which do not exist in nature but are recognized by the mRS to incorporate ncAAs. The terms engineered or modified tRNAs cover *iter alia* "stop-codon-adapted tRNAs".

Such engineered or modified tRNAs according to the invention are characterized by mutations of their nucleotide sequence of the tRNA, which then due to additional potential matches and/or mismatches when folding up into a 3D structure will change not only the structure but also improve or at least change the stability of the modified tRNA.

According to a further embodiment of the present invention the cold-OTS, the platform, but also the individualised mRS of the present invention show an even more surprising increase in catalytic efficacy, when used together with a modified tRNA. This modified tRNA is characterized by its DNA sequence (in 5`->3` direction), which derives from the *M. burtonii* tRNA but includes a T to G mutation at position 39 (SEQ ID No: 2), namely GGAGACTTGATCATGTAGATCGAACGGACTCTAAATCC**G**TTCAGCCGGGTTAGATTCCCGGAGTTTCCGCCA

According to a further embodiment the present invention also provides anticodon modified tRNA deriving from SEQ ID No.: 2 particularly suitable and astonishingly efficient when used for elongation of recombinant proteins or polypeptides by the mRS as described herein.

While already the mRS as claimed herein show a higher catalytic activity when compared with other or pre-known PylRS, this effect is only one part of the stunning results and possibilities achievable with the present invention. The efficiency of integration of amino acids with non-proteinogenic functional groups (ncAAs) can be also increased by providing higher amounts of suitable or modified tRNAs which can position such ncAAs at the site of interest.

For this, it is interesting and useful that the mRS according to the invention is capable of recognising, binding and integrating ncAAs into the newly synthesized polypeptide or protein guided and directed by a nucleotide triplet in the mRNA selected e.g. from the group of termination codons and also sense codons such as TAG, TAA, TGA, AGA, AGG, TCA and TTA. Such approach follows the concept of the "stop codon suppression", which is a particularly straightforward strategy to incorporate ncAAs into proteins, wherein the tRNA-synthetase and a stop-codon-adapted tRNA together enable the ribosomal incorporation of ncAAs as a consequence of a mutated tRNA anticodon.

Alternatively, the mRS according to the invention is also capable of recognising, binding and integrating ncAAs into the newly synthesized polypeptide or protein guided and directed by a nucleotide triplet in the mRNA selected e.g. from the group including all sense codons and modified sense codons, among such the following are preferred TTG (leucine), CTG (leucine), TCC (serine), ATA (isoleucine). In this approach, a codon-specific tRNA is engineered to direct ribosomal incorporation of a ncAA bound to such engineered tRNA to the defined position of said sense codon at the mRNA and thus, in the elongated AA sequence of a recombinant protein or polypeptide generated in a stochastic manner (stochastic incorporation of natural AA and ncAA).

According to further embodiments the invention provides vectors comprising a nucleic acid sequence encoding the tRNA synthetase mutants (mRS) according to the invention, which will be transcribed, translated, and synthesised upon transfection into a host cell.

Additionally, according to still further embodiments the invention provides vectors comprising the nucleic acid sequence encoding one or several copies of one or more tRNAs selected from homologous, heterologous and engineered tRNAs sequences, which will be synthesised upon transfection into a host cell.

Optionally, it is provided as a further embodiment that a suitable vector may carry both the mRS according to the invention and one or several copies of one or more tRNAs suitable for the incorporation of ncAAs.

In the context of the present description the term "vector" is understood as molecular tool introducing a genetic information into a host cell. Accordingly, in the context of the invention a vector is selected from the group of pro- and eukaryotic expression plasmids or expression systems, viral vectors and viral derived expression systems.

For more efficient protein production, the present invention provides as mentioned above, the `cold OTS' with the mRS and a selection of suitable modified tRNAs, both e.g. provided on suitable expression vectors, not only as an improved research tool but also as a platform for the production of recombinant proteins or peptides of choice.

According to a further embodiment the invention provides a platform for the generation, modification and production of recombinant proteins, wherein the recombinant proteins are generated by employing the cold-OTS as mentioned above, including the mRS of the invention and optionally, suitable modified tRNAs as mentioned above.

For this, the mRS and optionally, suitable and/or modified tRNAs, both encoded on one or several expression vectors, are introduced into a host cell culture together with an expression vector or an mRNA-shuttle carrying or capable of expressing the mRNA target sequence. The mRNA target sequence is to be understood as the matrices for generation of the recombinant polypeptide or protein to be synthesised, i.e. the matrices for a targeted and site-specific introduction and/or incorporation of ncAA into the ribosomal growing recombinant protein or polypeptide sequence.

The present invention thus enables further the use of the mRS as mentioned above alone or in combination with the modified tRNAs as mentioned above for the generation and/or production of recombinant polypeptides and/or protein incorporating in pre-determined and site-specific locations amino acids with non-proteinogenic functional groups introducing new functionalities to the synthetically generated proteins or polypeptides.

Many amino acids with non-proteinogenic functional groups (ncAAs) are already known in the art and some are illustrated in Figure 5a. To better understand such pre-existing knowledge, it might be interesting to consider photo-Leucine (Figure 6, 53).

The structure of photo-leucine closely resembles natural leucine. This similarity allows photo-leucine to be incorporated into proteins by the unmodified translation machinery of mammalian and bacterial cells (WO2006094745A1). Accordingly, in such pre-described approaches photo-leucine will be incorporated statistically at leucine positions throughout the proteome. Such method, however, requires that leucine is absent or just present in very low concentrations in the cultivation medium. This is not possible for all cells usually used, e.g. HEK293T cells because they die. For bacterial cells using a leucine-free medium results in drastically reduced cell numbers and concomitant low cell masses.

This already briefly describes the problems of the existing methods. Consequently, even if an incorporation of ncAAs into newly synthesized proteins seems possible, the presently existing methods are so inefficient that by no way a platform to produce recombinant proteins seems feasible. Additionally, the presently described methods are not suitable for a site-specific incorporation of an ncAA into a growing protein. Furthermore, no method was available to circumvent the native machinery with an exogeneous system for better then statistically incorporation of e.g. photo-leucine.

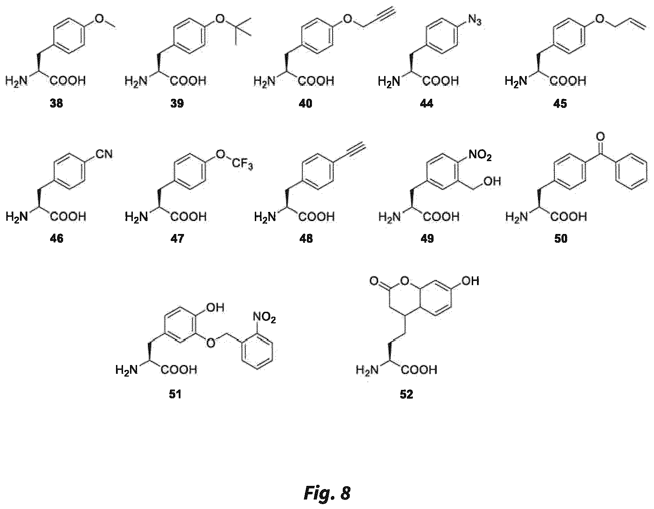

Interestingly, the inventors where able to demonstrate that with the mRS of the invention and accordingly the platform provided herein the incorporation of e.g. photo-leucine as well as other lysine derivatives, namely BocK (31), AllocK (32), ProK (33), AzidoK (34), PhotoK (42), BenzK (43) (as illustrated in Figure 8), can be incorporated in a much more efficient number than the statistical expectation and also can be targeted. Thus, the platform as described herein shows for the first time an OTS suitable for site-specific and highly efficient incorporation of Photo-leucine.

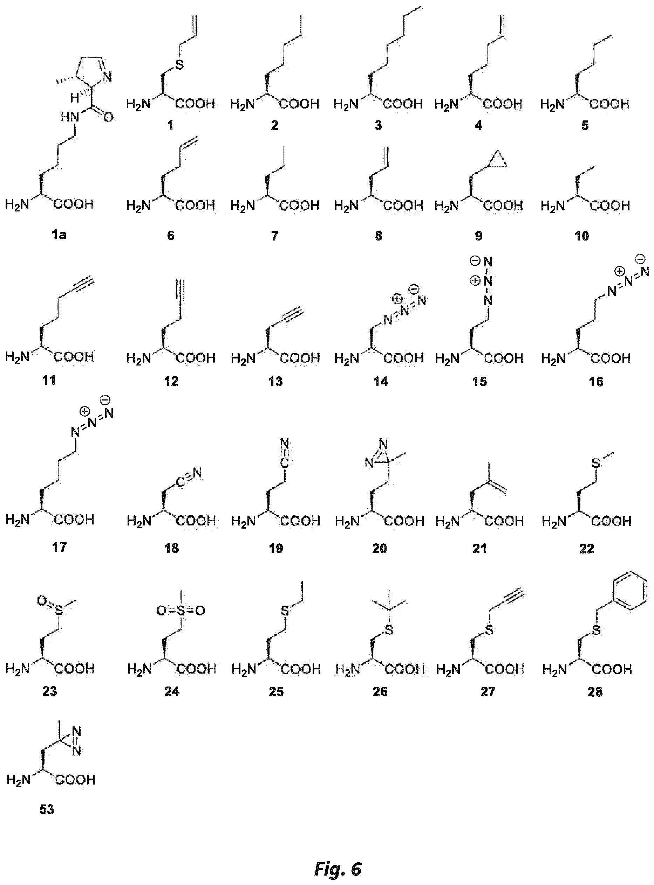

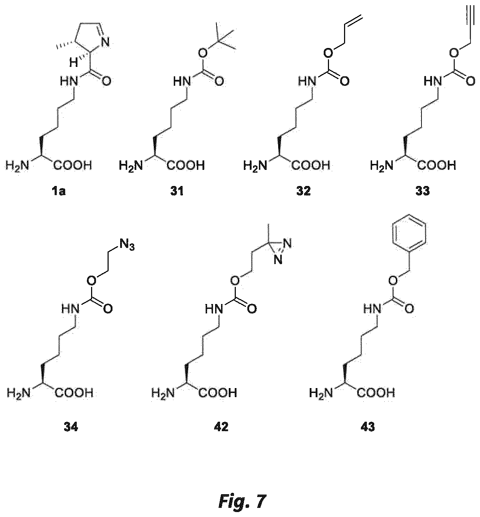

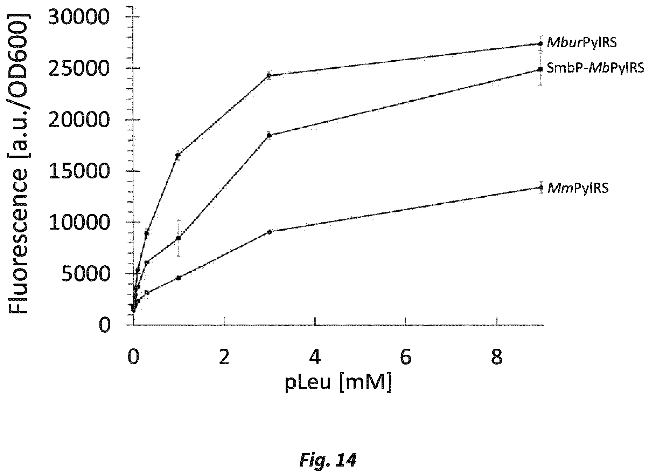

The inventors could show that the mRS of the invention can incorporate a high number of known incorporatable ncAAs with substantially higher efficiency. In this regard it was shown that beside the lysine derivatives (as of Figure 7) also other known ncAAs could be incorporated with much higher efficiency. Such other ncAAs used by the inventors in the various experiments are illustrated and identified by numbers in Figure 6 to 8. Particularly seen in the Figures 14, 17 and 18.

Furthermore, multiple installments e.g. of ncAAs S-allyl-L-cystein (1), *S*-propargyl-L-cystein (27), BocK (31), AllocK (32), or AzidoK (34) into recombinant proteins have proven to be more efficient with the mRS of the invention than with known PylRS systems. Particularly shown in the multiple or target specific integration of photo-leucine (Figure 12 to Figure 16).

In this regard it was also shown that when the corresponding tRNA anticodon is changed to address the TTG codon which encodes leucine, a statistical increased incorporation for ncAA (1) and also such statistical incorporation of photo-leucine (53) was obtainable. It is to remember that since the anticodon of the tRNA can be freely chosen, all sense-codons can be recoded for such statistical incorporation for a ncAA, e.g. photo-leucine.

In summary, it was - *iter alia* - found that the mRS of the present invention not only can incorporate ncAAs (1) and (27) but also photo-leucine (53) and many other ncAAs. Thus, this is the first system ever described which may increase the amount of incorporated ncAA e.g. photo-leucine and also allows or forces a site-specific incorporation of the ncAA of choice, e.g. photo-leucine (53).

Accordingly, the inventors have provided a system for the site-specific and residue specific incorporation of ncAA into recombinant proteins or polypeptides. Most advantageously, the method or OTS herein described does not rely on the use of any special cultivation medium with no or low concentrations of e.g. leucine, but empowers cell culture systems of all kind, which then can be grown in their preferred cultivation medium to ensure healthy proliferation.

The method or OTS herein described, thus not only allows to incorporate new functionalities but also can incorporate residues for coupling or conjugating moieties of choice to a given recombinant protein generated. Typically, moieties to be conjugated are selected from therapeutically or scientifically interesting peptides, proteins, targeting moieties, antibodies, nanobodies, cell determinants (CDs), receptors ligands, chemical conjugants, small molecules, pharmaceutically relevant drugs, radio-active isotopes.

The platform technology will be provided as a kit, including in suitable containers and/or buffers the mRS of the invention either as an enzyme ready-to-use or encoded on an expression vector, further including in suitable containers and/or buffers a selection of suitable tRNAs as mentioned above, optionally including in suitable containers and/or buffers a host cell system adapted to the mRS of the invention and further optionally, including in suitable containers and/or buffers an expression vector for the molecular modification or expression of an mRNA of choice. The kit will further include an instruction manual for the cold-OTS and the method of generating the recombinant proteins or polypeptides with new functionalities.

Said method of generating the recombinant proteins or polypeptides with new functionalities by the incorporation of amino acids with non-proteinogenic functional groups (ncAAs) into the elongated recombinant proteins comprises firstly the steps of providing, selecting or pre-modifying on a suitable vector or in a host cell the nucleic acid sequence, preferably the mRNA to be targeted for ncAA incorporation e.g by modifying triplets suitable for site specific incorporation. In a next step the method provides a vector encoding and expressing the mRS according to the invention. The method further includes a step of either transfecting, infecting and incubating a host cell culture with the preselected vectors under suitable cell culture conditions, during which the mRNA and the mRS are synthesised. In the most straight forward approach in the cold-OTS the mRS will bind the homologous tRNAs as of the host cell and by ribosomal elongation synthesise the recombinant protein.

As the mRS of the invention is much more efficient than previously known aaRS the production rate of the newly synthesised recombinant proteins is substantially increased (compare Figure 11, Figure 17, Figure 18). Also, the rate of ncAA incorporation by the mRS of the invention is much higher than found with known aaRS as the catalytic efficiency of the mRS of the invention and thus the rate of incorporation ncAA is clearly improved (Figure 12).

The present description also provides an even more elaborate method as a tool for synthetic biology, wherein - as described above - the mRS and the mRNA are provided in host cell system and additionally also heterologous and/or engineered tRNAs are introduced into the host cell system. Such one or several additional homologue tRNAs, heterology tRNAs and/or modified tRNAs as specified before are introduced into the host cell either on the same expression vectors as mRS and/or mRNA or alternatively, can be introduced as single or multiple copies of one or multiple tRNAs encoded on one or several separate expression vectors.

Similarly, as described above, in a first step a suitable vector for transfecting the mRNA to be used as matrix for the generation of the new recombinant protein is selected and introduced into the host cell system. Then in a next step, a vector encoding and expressing the mRS according to the invention, optionally, a vector encoding and expressing suitable tRNAs (heterologous tRNAs or modified/engineered tRNAs) and further optionally suitable ncAA for the incorporation into the newly synthesised polypeptide will be transfected, infected, and incubated with the host cell culture. In such suitable cell culture and under suitable cell culture conditions, both known to the practitioner from experience with said cell culture, the mRNA, the mRS and the tRNAs are transcribed and/or translated for then synthesising the new recombinant protein or polypeptide by ribosomal elongation.

As the platform is considered for the production of recombinant proteins or polypeptides with or without new functionalities the method as a further step comprises the isolation and purification of the synthesised recombinant protein from the host cells, the supernatant and/or the host cell culture.

According to further embodiment the host cells for the above-described platform and cold-OTS are of prokaryotic or eukaryotic origin. Suitable cell cultivation conditions are known to the practitioner. The higher efficiency of the "cold"-OTS based on the mRS of the invention also holds true in various hosts, and thus can lead to improved and expanded applicability for the production of host adapted therapeutically interesting, but also non-therapeutically relevant recombinant proteins.

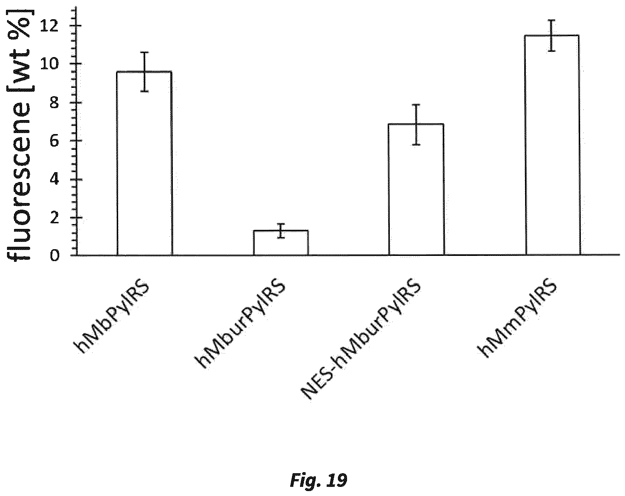

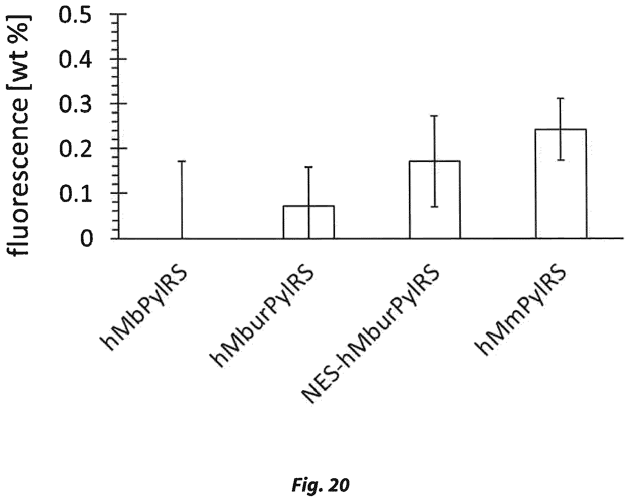

Additionally, and according to further embodiments the mRS can be enhanced by additionally cloning a nuclear export signal (NES) onto the mRS coding sequence. One way to incorporate such nuclear export signal is to clone the NES right after the starting codon (ATG) into the coding sequence of the mRS, thereby creating a fusion between the NES and mRS. Known NES are suitable for all described mRS of the present invention. According to the present invention one preferred NES is the codon optimised sequence (SEQ ID No:11) "5-GCTTGTCCTGTTCCTCTGCAGCTGCCTCCTCTGGAAAGACTGACCCTGGAT-3". The typical increase regarding the *in vivo* efficiency is described in Example 7 together with Figure 19 and 20.

The present invention is not only advantageous for producing new recombinant proteins and recombinant proteins with new functionalities but is also particularly interesting for basic science of protein-protein interactions. Such protein-protein interactions are the key to organizing cellular processes in space and time. One direct way, which is thanks to the improvements of the invention now accessible for scientists, is to identify such interactions in their cellular environment is by e.g. photo-cross-linking. Photo-Leucine is especially useful for that matter because it is a "zero-length" cross-linker minimizing false positive cross-link-interactions.

| SEQ ID NO: | Residues |
|---|---|
| 1 | MEKQLLDVLVELNGVWLSRSGLLHGIRNFEITTKHIHIETDCGARFTVRNSRSSRSAR SLRHNKYRKPCKRCRPADEQIDRFVKKTFKEKRQTVSVFSSPKKHVPKKPKVAVIKS FSISTPSPKEASVSNSIPTPSISVVKDEVKVPEVKYTPSQIERLKTLMSPDDKIPIQDEL PEFKVLEKELIQRRRDDLKKMYEEDREDRLGKLERDITEFFVDRGFLEIKSPIMIPFEYI ERMGIDKDDHLNKQIFRVDESMCLRPMLAPCLYNYLRKLDKVLPDPIRIFEIGPCYRK ESDGSSHLEEFTMVXFXQMGSGCTRENMEALIDEFLEHLGIEYEIEADNCMVXGDTID IMHGDLELSSAVVGPIPLDREWGVNKPXMGAGFGLERLLKVRHNYTNIRRASRSELY YNGINTNL |
| Consensus mRS | |
| 2 | GGAGACTTGATCATGTAGATCGAACGGACTCTAAATCCGTTCAGCCGGGTTAGATT CCCGGAGTTTCCGCCA |
| modified tRNA | |
| 3 | MEKQLLDVLVELNGVWLSRSGLLHGIRNFEITTKHIHIETDCGARFTVRNSRSSRSAR SLRHNKYRKPCKRCRPADEQIDRFVKKTFKEKRQTVSVFSSPKKHVPKKPKVAVIKS FSISTPSPKEASVSNSIPTPSISVVKDEVKVPEVKYTPSQIERLKTLMSPDDKIPIQDEL PEFKVLEKELIQRRRDDLKKMYEEDREDRLGKLERDITEFFVDRGFLEIKSPIMIPFEYI ERMGIDKDDHLNKQIFRVDESMCLRPMLAPCLYNYLRKLDKVLPDPIRIFEIGPCYRK ESDGSSHLEEFTMVNFCQMGSGCTRENMEALIDEFLEHLGIEYEIEADNCMVFGDTI DIMHGDLELSSAVVGPIPLDREWGVNKPWMGAGFGLERLLKVRHNYTNIRRASRSEL YYNGINTNL |
| mRS Y346 F | |
| 4 | MEKQLLDVLVELNGVWLSRSGLLHGIRNFEITTKHIHIETDCGARFTVRNSRSSRSAR SLRHNKYRKPCKRCRPADEQIDRFVKKTFKEKRQTVSVFSSPKKHVPKKPKVAVIKS FSISTPSPKEASVSNSIPTPSISVVKDEVKVPEVKYTPSQIERLKTLMSPDDKIPIQDEL PEFKVLEKELIQRRRDDLKKMYEEDREDRLGKLERDITEFFVDRGFLEIKSPIMIPFEYI ERMGIDKDDHLNKQIFRVDESMCLRPMLAPCLYNYLRKLDKVLPDPIRIFEIGPCYRK ESDGSSHLEEFTMVAFAQMGSGCTRENMEALIDEFLEHLGIEYEIEADNCMVFGDTID IMHGDLELSSAVVGPIPLDREWGVNKPWMGAGFGLERLLKVRHNYTNIRRASRSELY YNGINTNL |
| mRS | |
| N308A:C310A:Y346F | |
| 5 | MEKQLLDVLVELNGVWLSRSGLLHGIRNFEITTKHIHIETDCGARFTVRNSRSSRSAR SLRHNKYRKPCKRCRPADEQIDRFVKKTFKEKRQTVSVFSSPKKHVPKKPKVAVIKS FSISTPSPKEASVSNSIPTPSISVVKDEVKVPEVKYTPSQIERLKTLMSPDDKIPIQDEL PEFKVLEKELIQRRRDDLKKMYEEDREDRLGKLERDITEFFVDRGFLEIKSPIMIPFEYI ERMGIDKDDHLNKQIFRVDESMCLRPMLAPCLYNYLRKLDKVLPDPIRIFEIGPCYRK ESDGSSHLEEFTMVGFGQMGSGCTRENMEALIDEFLEHLGIEYEIEADNCMVFGDTI DIMHGDLELSSAVVGPIPLDREWGVNKPWMGAGFGLERLLKVRHNYTNIRRASRSEL YYNGINTNL |
| mRS | |
| N308G:C310G:Y346F | |
| 6 | MEKQLLDVLVELNGVWLSRSGLLHGIRNFEITTKHIHIETDCGARFTVRNSRSSRSAR SLRHNKYRKPCKRCRPADEQIDRFVKKTFKEKRQTVSVFSSPKKHVPKKPKVAVIKS FSISTPSPKEASVSNSIPTPSISVVKDEVKVPEVKYTPSQIERLKTLMSPDDKIPIQDEL PEFKVLEKELIQRRRDDLKKMYEEDREDRLGKLERDITEFFVDRGFLEIKSPIMIPFEYI ERMGIDKDDHLNKQIFRVDESMCLRPMLAPCLYNYLRKLDKVLPDPIRIFEIGPCYRK ESDGSSHLEEFTMVMFWQMGSGCTRENMEALIDEFLEHLGIEYEIEADNCMVFGDTI DIMHGDLELSSAVVGPIPLDREWGVNKPWMGAGFGLERLLKVRHNYTNIRRASRSEL YYNGINTNL |
| mRS | |
| N308M:C310W:Y346F | |
| 7 | MEKQLLDVLVELNGVWLSRSGLLHGIRNFEITTKHIHIETDCGARFTVRNSRSSRSAR SLRHNKYRKPCKRCRPADEQIDRFVKKTFKEKRQTVSVFSSPKKHVPKKPKVAVIKS FSISTPSPKEASVSNSIPTPSISVVKDEVKVPEVKYTPSQIERLKTLMSPDDKIPIQDEL PEFKVLEKELIQRRRDDLKKMYEEDREDRLGKLERDITEFFVDRGFLEIKSPIMIPFEYI ERMGIDKDDHLNKQIFRVDESMCLRPMLAPCLYNYLRKLDKVLPDPIRIFEIGPCYRK ESDGSSHLEEFTMVQFWQMGSGCTRENMEALIDEFLEHLGIEYEIEADNCMVFGDTI DIMHGDLELSSAVVGPIPLDREWGVNKPWMGAGFGLERLLKVRHNYTNIRRASRSEL YYNGINTNL |
| mRS | |
| N308Q:C310W:Y346F | |
| 8 | MEKQLLDVLVELNGVWLSRSGLLHGIRNFEITTKHIHIETDCGARFTVRNSRSSRSAR SLRHNKYRKPCKRCRPADEQIDRFVKKTFKEKRQTVSVFSSPKKHVPKKPKVAVIKS FSISTPSPKEASVSNSIPTPSISVVKDEVKVPEVKYTPSQIERLKTLMSPDDKIPIQDEL PEFKVLEKELIQRRRDDLKKMYEEDREDRLGKLERDITEFFVDRGFLEIKSPIMIPFEYI ERMGIDKDDHLNKQIFRVDESMCLRPMLAPCLYNYLRKLDKVLPDPIRIFEIGPCYRK ESDGSSHLEEFTMVLFWQMGSGCTRENMEALIDEFLEHLGIEYEIEADNCMVFGDTI DIMHGDLELSSAVVGPIPLDREWGVNKPWMGAGFGLERLLKVRHNYTNIRRASRSEL YYNGINTNL |
| mRS | |
| N308L:C310W:Y346F | |
| 9 | MEKQLLDVLVELNGVWLSRSGLLHGIRNFEITTKHIHIETDCGARFTVRNSRSSRSAR SLRHNKYRKPCKRCRPADEQIDRFVKKTFKEKRQTVSVFSSPKKHVPKKPKVAVIKS FSISTPSPKEASVSNSIPTPSISVVKDEVKVPEVKYTPSQIERLKTLMSPDDKIPIQDEL PEFKVLEKELIQRRRDDLKKMYEEDREDRLGKLERDITEFFVDRGFLEIKSPIMIPFEYI ERMGIDKDDHLNKQIFRVDESMCLRPMLAPCLYNYLRKLDKVLPDPIRIFEIGPCYRK ESDGSSHLEEFTMVNFWQMGSGCTRENMEALIDEFLEHLGIEYEIEADNCMVFGDTI DIMHGDLELSSAVVGPIPLDREWGVNKPTMGAGFGLERLLKVRHNYTNIRRASRSEL YYNGINTNL |
| mRS | |
| C310W:Y346F:S379T | |
| 10 | ATGGAAAAGCAGCTCCTGGACGTGCTGGTGGAACTGAATGGCGTGTGGCTGAGCA GATCTGGCCTGCTGCACGGCATCCGGAACTTCGAGATCACCACCAAGCACATCCA CATCGAGACAGACTGCGGCGCCAGATTCACCGTGCGGAACAGCAGATCTAGCAGA AGCGCCAGAAGCCTGCGGCACAACAAGTACAGAAAGCCCTGCAAGAGATGCAGAC CCGCCGACGAGCAGATCGACAGATTCGTGAAGAAAACCTTCAAAGAAAAGCGGCA GACCGTGTCCGTGTTCAGCAGCCCCAAGAAACACGTGCCCAAAAAGCCCAAGGTG GCCGTGATCAAGAGCTTCAGCATCAGCACCCCATCTCCAAAAGAGGCCAGCGTCA GCAACAGCATCCCTACACCTAGCATCAGCGTGGTCAAGGACGAAGTGAAGGTGCC AGAAGTGAAGTACACCCCTAGCCAGATCGAGCGGCTGAAAACCCTGATGAGCCCC GACGACAAGATCCCCATCCAGGATGAGCTGCCCGAGTTCAAGGTGCTGGAAAAAG AGCTGATCCAGCGGCGGAGGGACGACCTGAAGAAGATGTACGAAGAGGACAGAGA GGACCGGCTGGGCAAGCTGGAAAGAGACATCACCGAGTTCTTCGTGGACCGGGG CTTCCTGGAAATCAAGAGCCCCATCATGATCCCGTTCGAGTACATCGAGCGGATG GGCATCGACAAGGACGACCACCTGAACAAGCAGATTTTCAGAGTGGACGAGAGCA TGTGCCTGCGGCCTATGCTGGCCCCTTGCCTGTACAACTACCTGCGGAAGCTGGA TAAGGTGCTGCCCGATCCTATCCGCATCTTTGAGATCGGCCCCTGCTACCGGAAA GAGAGCGACGGATCTAGCCACCTGGAAGAGTTCACCATGGTCAATTTCTGGCAGA TGGGCAGCGGCTGCACCCGCGAGAATATGGAAGCCCTGATCGACGAGTTCCTGGA ACACCTGGGAATCGAGTACGAGATCGAGGCCGACAACTGCATGGTGTTCGGCGAC ACCATCGACATCATGCACGGCGACCTGGAACTGTCTAGCGCCGTTGTGGGACCCA TTCCTCTGGATAGAGAATGGGGCGTGAACAAGCCCACAATGGGAGCCGGCTTTGG CCTGGAAAGACTGCTGAAAGTGCGCCACAACTACACCAACATCAGACGGGCCAGC CGCAGCGAGCTGTACTACAACGGCATCAACACCAACCTGTGA |
| h-mRS | |
| C310W:Y346F:W379T | |
| 11 | GCTTGTCCTGTTCCTCTGCAGCTGCCTCCTCTGGAAAGACTGACCCTGGAT |
| NES codon optimised | |

### Short description of the Figures:

**Figure 1** shows the collinearity of the coding nucleotide sequences of DNA and mRNA and the amino acid sequence of a polypeptide chain, adapted. Not shown is the deoxyribose and phosphate backbone
**Figure 2** shows according to Saier (2019) **A**) Codon wheel of the genetic code in RNA codons with cAA abbreviated in the three letter code. The total of 64 RNA triplets are assigned 61 sense-codons for 20 canonical amino acids and three nonsense-codons (Ter). The 5'-to-3' direction is read from the inside to the outside and indicates the first, second and third position of the codon. B) Rearranged codon wheel in which the second codon position is clustered, highlighting its importance.
**Figure 3** shows extracted from Stryer et al. "Biochemistry", 2015*.* Two-dimensional depiction of a generic tRNA in the typical cloverleaf structure. The tRNA generally contains four characteristic loops. The dihydrouracil loop (DHU loop), the anticodon loop, the extra arm (variable loop, which contains a variable number of bases) and the T_{ψ}C loop (containing ribothymidine (T), pseudouridine(Ψ) and cytosine, hence the name). **B**) Three-dimensional structure of tRNA. The four double-stranded regions of tRNA stack to form an L-shaped structure.
**Figure 4** shows a schematic representation of the protein biosynthesis machinery with an introduced orthogonal translation system: 1) Specific activation of the ncAA with the orthogonal aaRS within a pool of canonical AAs. 2) Binding of corresponding orthogonal tRNA to orthogonal aaRS ("loading" of the orthogonal tRNA with ncAA). 3) Aminoacylated orthogonal tRNA (ncAA-otRNA) leaves the orthogonal aaRS. 4) Binding of the elongation factor TU (EF-TU). 5) Binding to the aminoacyl site of the ribosome (A site). There, the anticodon of the tRNA forms hydrogen bridge bonds to the corresponding mRNA codon. Then at the peptidyl site (P site), the tRNA transfers the nascent polypeptide chain (NPC) to the ncAA-otRNA to elongate the NPC by the additional ncAA. After transfer, the tRNA at the P site moves to the exit site (E site) and leaves the ribosome. Translation ends when a stop-codon arrives at the A site. At that point, the release factor binds to the ribosome and initiates the release of the polypeptide chain.

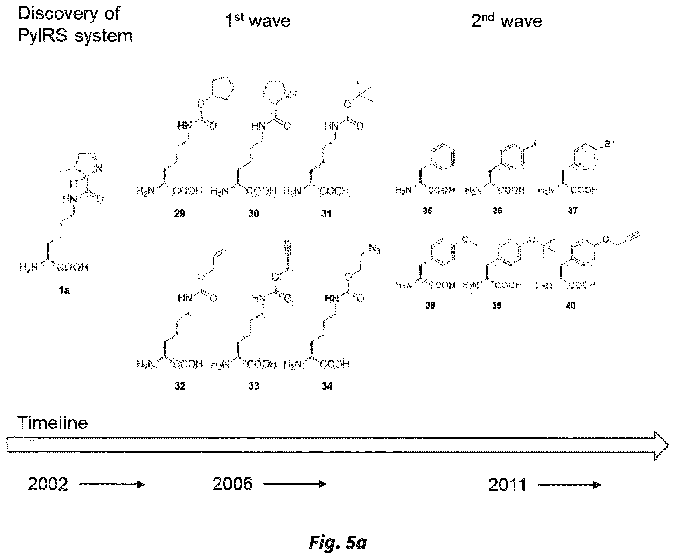

**Figure 5a** shows a flowchart of the discovery and timetable of characteristic ncAAs that can be incorporated with previously described the OTS.

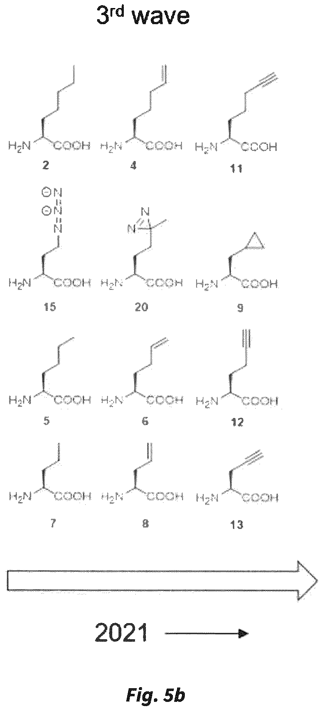

**Figure 5b****:** The third wave refers to ncAAs which have been successfully incorporated with improved efficiency of the mRA in the present invention.
**Figure 6** shows amino acids used in this description.
   Chemical structure of pyrrolysine (**1a**), S-allyl-L-cystein (**1**), (*S*)-2-aminoheptanoic acid (**2**), (*S*)-2-aminooctanoic acid (**3**), (*S*)-2-aminohept-6-enoic acid (**4**), (*S*)-2-aminohexanoic acid (**5**), (*S*)-2-aminohex-5-enoic acid (**6**), (*S*)-2-aminopentanoic acid (**7**), (*S*)-2-aminopent-4-enoic acid (**8**), (*S*)-2-amino-3-cyclopropylpropanoic acid (**9**), (*S*)-2-aminobutyric acid (**10**), (*S*)-2-aminohept-6-ynoic acid (**11**), (*S*)-2-aminohex-5-ynoic acid (**12**), (*S*)-2-aminopent-4-ynoic acid (**13**), (*S*)-2-amino-3-azidopropanoic acid (**14**), (S)-2-amino-4-azidobutanoic acid (**15**), (*S*)-2-amino-5-azidopentanoic acid (**16**), (*S*)-2-amino-6-azidohexanoic acid (**17**), (*S*)-2-amino-3-cyanopropanoic acid (**18**), (*S*)-2-amino-4-cyanobutanoic acid (**19**), (*S*)-2-amino-5,5'-azi-hexanoic acid (**20**), (*S*)-2-amino-4-methylpent-4-enoic acid (**21**), L-methionine (**22**), L-methionine sulfoxide (**23**), L-methionine sulfone (**24**), L-ethionine (**25**), *S*-tert-butyl-L-cysteine (**26**), *S*-propargyl-L-cysteine (**27**), *S*-benzyl-L-cysteine (**28**), photo-leucine (pLeu, **53**).
**Figure 7** shows: Pyrrolysine (**1a**) and derivatives used in this description.
   BocK = *N*^{ε}-*tert*-Butoxycarbonyl-L-lysine (**31**), AllocK = *N*^{ε}-Allyloxycarbonyl-L-lysine (**32**),
   ProK = *N*^{ε}-Propargyloxycarbonyl-L-lysine (**33**), AzidoK = *N*^{ε}-((2-Azidoethoxy)carbonyl)-L-lysine (**34**), PhotoK = 3'-Azibutyl-*N* -carbamoyl-L-lysine (**42**), BenzK = *N* -benzyloxycarbonyl-L-lysine (**43**).
**Figure 8** shows Phe/Tyr derivatives used in this description. O-methyl-Y = *O*-methyl-L-tyrosine (**38**), O-tert-butyl-Y = *O*-*tert*-butyl-L-tyrosine (**39**), O-prop-Y = *O*-propargyl-L-tyrosine (**40**), azido-F = 4-azido-L-phenylalanine (**44**), O-allyl-Y = *O*-allyl-L-tyrosine (**45**), cyano-F = 4-cyano-L-phenylalanine (**46**), O-CF₃-Y = *O*-CF₃-L-tyrosine (**47**), ethynyl-F = 4-ethynyl-L-phenylalanine (**48**), p-oNB-alanine (**49**), Bpa = 4-benzoyl-L-phenylalanine (**50**), *m*-oNB-Dopa = *o*-(2-Nitrobenzyl)-3,4-dihydroxyphenylalanine (**51**), coumarin = H-(7-Hydroxycoumarin-4-yl)-ethyl-Gly-OH (**52**).
**Figure 9** shows *M. burtonii* and *M. alaskense* tRNA^{Pyl}. ΔG values in brackets, calculated at 37 °C. The nucleotide binding probability is indicated by color; red = high, green = middle and blue = low. The fold and free energy prediction was performed with Geneious which uses the ViennaRNA Package. The nucleotide with blue a circle is the 5' end and with a red circle the 3' end.
**Figure 10** shows the concentration-dependent unnatural protein production with *Mbur*PylRS and *Mala*PylRS for different tRNA (organism abbreviation)/ncAA combinations. Using *E*. *coli* BL21(DE3) cells and SUMO-sfGFP (1x amber) as reporter. Endpoint measurements for ncAA concentrations of 0.025, 0.05, 0.1, 0.3, 1, 3 and 9 mM, Gain 85.
**Figure 11** shows the concentration-dependent unnatural protein production with **A**) MjONB-DopaRS with substrate **51, B**) *Mj*ONBYRS with substrate O-Nitrobenzene-Tyrosine, **C**) *Mbur*PylRS with tRNAfrom *M. burtonii* and substrate **31, D**) MburPylRS(C310W:Y346F:W379T) with tRNA from *M. burtonii* and substrate **1, E**) *Mbur*PylRS with tRNA from *M. alaskense* and substrate **31,** and **F**) MburPylRS(C310W:Y346F:W379T) with tRNA from *M. alaskense* and substrate **1.** The host for protein production was RF1 deficient *Escherichia coli* B-95.ΔA. Endpoint measurements for ncAA concentrations of 0.025, 0.05, 0.1, 0.3, 1 and 3 mM. Fluorescence values were normalized to values of wild-type sfGFP reporter constructs (without an in-frame stop codon).

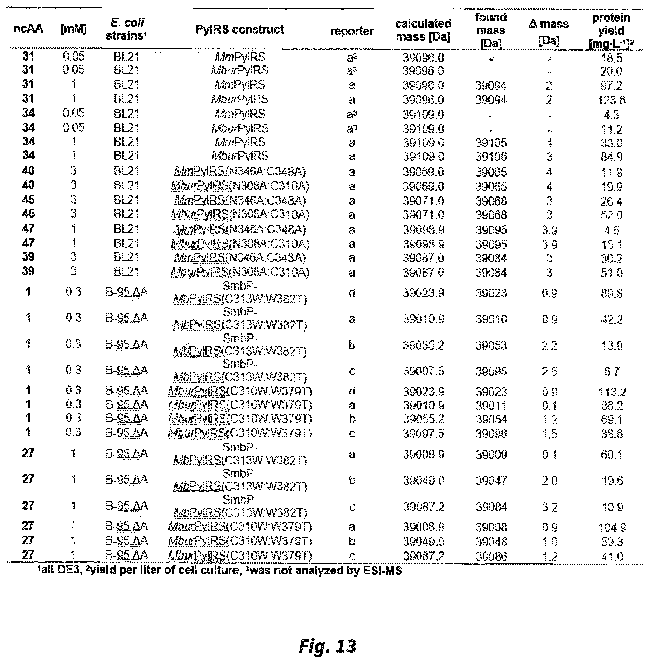

**Figure 12** shows the concentration-dependent unnatural protein production with *Mm*PylRS (**A, C**) and mRS *Mbur*PylRS (**B, D**) for different ncAA/reporter construct combinations. The host for protein production was *E. coli* B-95. ΔA. Endpoint measurements for ncAA concentrations of 0.025, 0.05, 0.1, 0.3,1, 3 and 9 mM.**Figure 13** shows the protein production with calculated and observed molecular weights of reporter proteins. Setup of protein production platform with calculated and observed molecular weights of reporter proteins corresponding to a) SUMO-sfGFP(1x amber), b) SUMO-sfGFP(3x amber), c) SUMO-sfGFP(5x amber), d) SUMO-sfGFP(wild-type). The masses were determined by ESI-MS of the intact proteins. In the Figure itself the legend is shortend: all mentioned mRS contain the amino acid exchange Y346F, nevertheless only additional exchanges are explicitly mentioned on the figure.
**Figure 14** shows the concentration-dependent unnatural protein production of SUMO-sfGFP(1x amber) with mRS MburPylRS(C310W:Y346F:W379T), SmbP-*Mb*PylRS(C313W:Y346F:W382T) and *Mm*PylRS(C348W:Y346F:W417T) with supplied photo-leucine (**53**). The host for protein production was *E. coli* BL21(DE3). Endpoint measurements for ncAA concentrations of 0.025, 0.05, 0.1, 0.3,1, 3 and 9 mM.

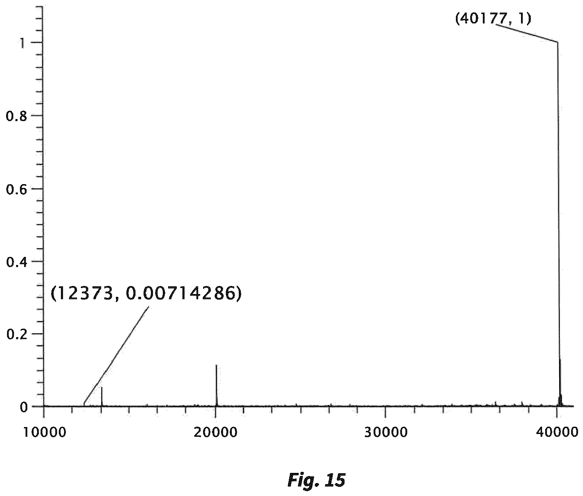

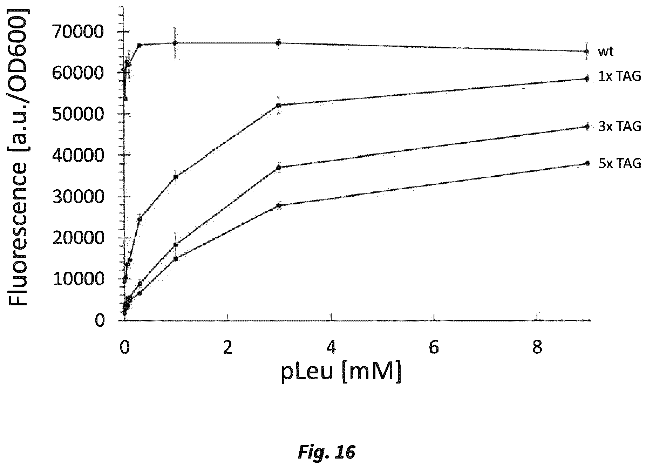

**Figure 15** shows the deconvoluted ESI-MS spectrum of His₆-SUMO-sfGFP(R2(photo-leucine))-Strep production in *E*. *coli* BL21(DE3) with co-expression of mRS *Mbur*PylRS (C310W:Y346F:W379T). Expected protein mass: 40176.8 Da. Observed mass: 40177 Da. Expected mass of His₆-SUMO truncation product: 12372.8 Da. Observed mass: 12372 Da**Figure 16** shows the endpoint measurements of concentration-dependent unnatural protein production (SUMO-sfGFP) with different number of stop codons (amber = TAG) and the wild type (wt) without and in-frame stop codon. The mRS used was *Mbur*PylRS(C310W:Y346F:W379T) with tRNA from *M. alaskense* and photo-leucine supplied in different concentrations (0.025,0.05,0.1,0.3,1,3 and 9 mM). The host for protein production was B-95. ΔA.**Figure 17** shows: Heatmaps based on the fluorescence of the sfGFP (1x amber) expression with E. *coli* BL21(DE3) for the eight selected PylRS (mRS included) in dependence of different ncAA concentrations; **A), B), C), D), E), F)** are 0.05, 0.1, 0,3, 1, 3, 9 mM ncAA supplied. Bar charts showing the number of replicates and error bars showing standard deviation are provided in the supplements. Substrate numbering is found in Figure 7. For all Figures the background suppression was construct dependent between 900 and 1,300 [a.u.] and subtracted from the values with supplied ncAA.

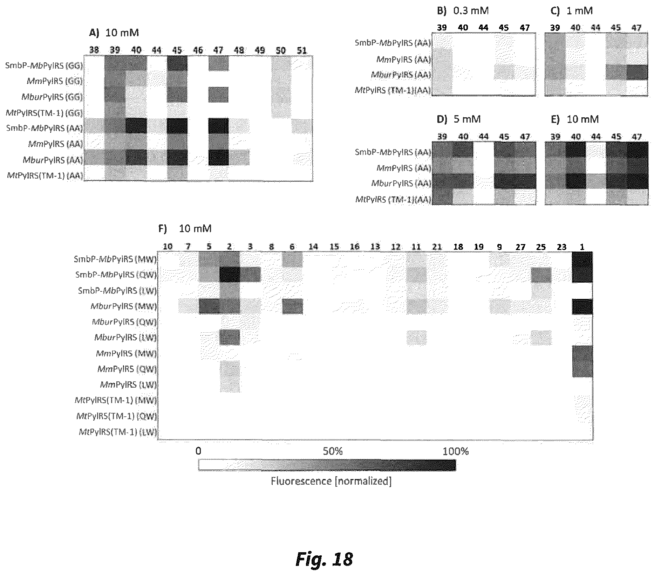

**Figure 18** shows: Heatmaps based on the fluorescence of the sfGFP(1x amber) expression with E. *coli* BL21(DE3). **A**) and **F**) are mRS (from *M. burtonii*) and PylRS mutants from other organisms with 10 mM supplied ncAA. Concentration dependent ncAA incorporation are **B), C), D), E)** with 0.3, 1, 5 and 10 mM ncAA supplied. Letters in brackets indicate the mutations in *M. border;* notation. First letter specifies the mutation at position N311 and the second letter at C313. Substrate numbering is found in Figure 1. Growth for *Mm*PylRS and *Mt*PylRS(TM-1) constructs and substrate **27** was abnormal low so these values were excluded. For all Figures the background suppression was construct dependent between 1,100 and 2,500 [a.u.] and subtracted from the values with supplied ncAA.
**Figure 19** shows: Fluorescence measurement of EGFP(Y183 amber) reporter protein production with HEK293T cells transfected with sequence humanized mRS and other PylRS constructs and 0.45 mM Sac (**1**) supplied. One mRS additionally contains a nuclear export sequence (NES).
**Figure 20** shows: Fluorescence measurement of EGFP(Y183 amber) reporter protein production with HEK293T cells transfected with sequence humanized mRS and other PylRS constructs and 0.5mM photo-leucine (**53**) supplied. One mRS additionally contains a nuclear export sequence (NES).

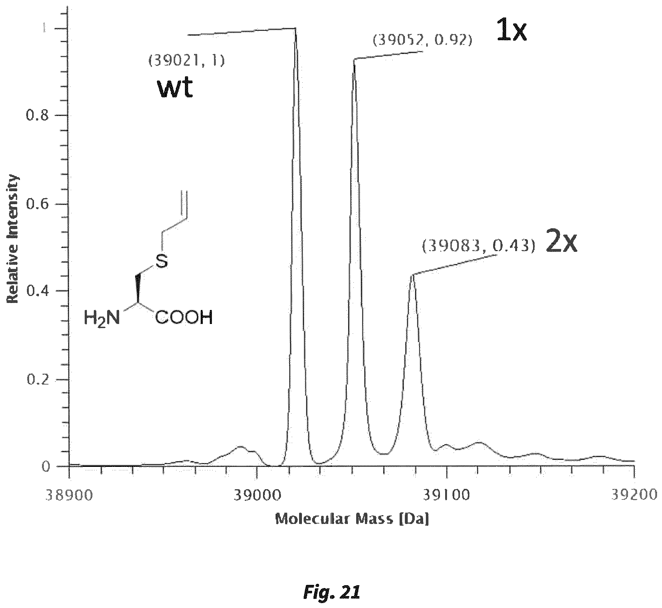

**Figure 21** shows: The deconvoluted ESI-MS spectrum of sfGFP with stochastic incorporation of Sac (**1**) targeting the TTG (Leu) codon in *E*. *coli* BL21(DE3) cells. The sfGFP gene contains two TTG leucine codons. Incorporation of one or two Sac (**1**) moieties is observable.

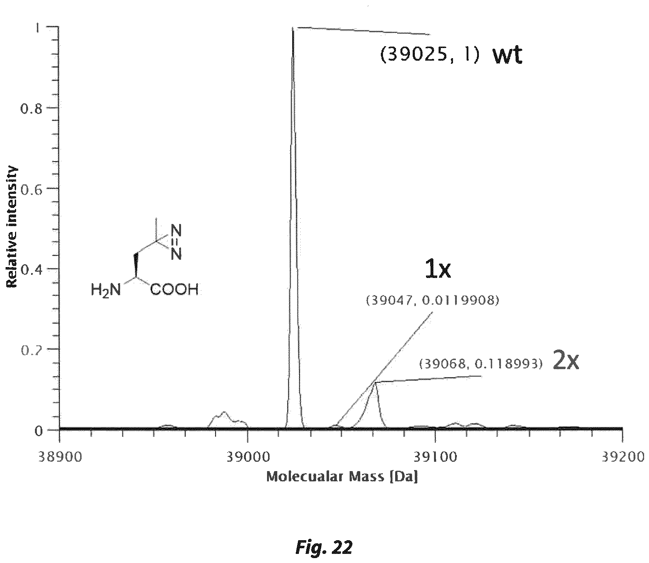

**Figure 22** shows: The deconvoluted ESI-MS spectrum of sfGFP with stochastic incorporation of photo-leucine (**53**) targeting the TTG (Leu) codon in *E*. *coli* BL21(DE3) cells. The sfGFP gene contains two TTG leucine codons. The sfGFP gene contains two TTG leucine codons. Incorporation of one or two photo-leucine (**53**) moieties is observable.

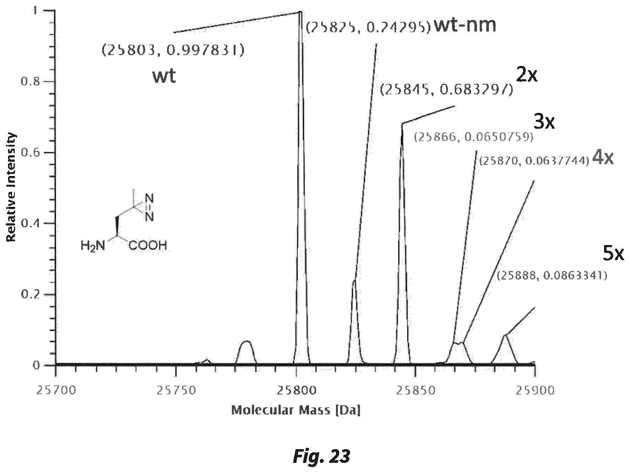

**Figure 23** shows: The Deconvoluted ESI-MS spectrum of amilCP with stochastic incorporation of photo-leucine (**53**) targeting the ATA (Ile) codon in *E*. *coli* BL21(DE3) cells. The amilCP gene contains codons coding for isoleucine. Incorporation of up to five photo-leucine (**53**) moieties is observable. The wt-nm peak is non-maturated amilCP.

### Examples

### Example 1: Generation, Introduction of mutations and Selection for suitable MburPylRS mutants

The crystal structures of MmPylRS (PDB ID 2Q7H) and the MmOmeRS (PDB ID 3QTC) mutant guided the rational mutation approach to incorporate all the small aliphatic ncAAs and other ncAAs as well.

For this all plasmids were assembled by Golden Gate cloning and confirmed by DNA sequencing. Point mutations were introduced by non-overlapping inverse PCR. Focused *Mbur*PylRS gene libraries were created also with non-overlapping inverse PCR, but randomization was performed using mutagenic primers (with NNK (N = A, T, G, or C; K = G or T)) at designated positions.

After library creation and transformation, 96 clones were picked and grown overnight in a 96-well plate in 100 µL LB with 1% glucose and appropriate antibiotics. The next day a 96-well plate with 100 µL ZYP 5052, appropriate antibiotics, and ncAAs was inoculated with 1 µL culture, grown for 24 h and measured afterwards as stated above. The 96-well plate which was used for inoculation was sealed with aluminum foil and stored at 4 °C. From this plate, desired clones were analyzed via PCR gene amplification and sequencing of this PCR product afterwards.

For the small-scale expression of fluorescence reporter constructs, *E. coli* cells were used. Electrocompetent cells were transformed with the orthogonal translation system and reporter plasmids. LB agar plates for plating contained 1 % glucose and corresponding antibiotics. Single colonies of clones were used for inoculation of 2 mL LB (in 14 mL tubes) with 1 % glucose and appropriate antibiotics and grown to saturation overnight. Assays were conducted in 96-well plate format. Cultures were added to each well at 1:100 dilution in ZYP-5052 auto induction medium to a final volume of 100 µL supplemented with antibiotics and ncAAs. Cells were grown in black µ-plates (Greiner Bio-One, Kremsmünster, Austria) covered with a gas permeable foil (Breathe-Easy^{®}, (Diversified Biotech, Doylestown, PA)) with orbital shaking for 24 h at 37 °C. For endpoint measurements (Tecan M200), the plate foil was removed, and fluorescence measured with an 85 gain setting. For OD₆₀₀ measurements, 50 µL of ZYP-5052 medium was introduced into clear 96-well µ-plates and 50 µL of culture added. Excitation and emission wavelengths for fluorescence measurements were set to 481 nm and 511 nm, respectively. Fluorescence values were normalized to the corresponding OD₆₀₀. Biological triplicates were used for measurements of each aaRS construct. Relative fluorescence was normalized to the highest value. The data (incl. standard deviation) represents the mean of three biological replicates

As a result of this method, one obtains the intact cell fluorescence which correlates with the incorporation efficiency of the used OTS (see as example Figure 10, 11, 12).

For the first time it was shown that a psychrophilic mutant PylRS of *M. burtonii* is functional in a bacterial cell host.

### Example 2: Testing MburPylRS (mRS) in comparison with other PylRS

By performing a comparative study for PylRS variants with extremophilic origins it was found that *Mbur*PylRS exhibits a remarkable ncAA incorporation efficiency especially at very low ncAA concentrations (Figure 17).

For this, all selected genes were assembled into plasmids by Golden Gate cloning and confirmed by DNA sequencing. Afterwards the ncAA incorporation efficiency was determined by intact cell fluorescence measurements, *E*. *coli* cells were used. Electrocompetent cells were transformed with the orthogonal translation system and reporter plasmids. LB agar plates for plating contained 1 % glucose and corresponding antibiotics. Single colonies of clones were used for inoculation of 2 mL LB (in 14 mL tubes) with 1 % glucose and appropriate antibiotics and grown to saturation overnight. Assays were conducted in 96-well plate format. Cultures were added to each well at 1:100 dilution in ZYP-5052 auto induction medium to a final volume of 100 µL supplemented with antibiotics and ncAAs. Cells were grown in black µ-plates (Greiner Bio-One, Kremsmünster, Austria) covered with a gas permeable foil (Breathe-Easy^{®}, (Diversified Biotech, Doylestown, PA)) with orbital shaking for 24 h at 37 °C. For endpoint measurements (Tecan M200), the plate foil was removed, and fluorescence measured with an 85 gain setting. For OD₆₀₀ measurements, 50 µL of ZYP-5052 medium was introduced into clear 96-well µ-plates and 50 µL of culture added. Excitation and emission wavelengths for fluorescence measurements were set to 481 nm and 511 nm, respectively. Fluorescence values were normalized to the corresponding OD₆₀₀. Biological triplicates were used for measurements of each aaRS construct. Relative fluorescence was normalized to the highest value. The data (incl. standard deviation) represents the mean of three biological replicates

The *Mbur*PylRS shows superior OTS efficiency for any substrate especially at low ncAA concentrations. This was observed for all major substrate classes known for the PylRS system, highlighting the robustness of the results (Figure 18). Most impressive was the multi-site ncAA incorporation with BocK (**2**) and especially Sac (**38**). To the best of our knowledge, no PylRS mutant has ever been reported to have nearly the same ncAA incorporation efficiency as the wild-type.

### Example 3: Comparison of MburPylRS mutants to state-of-the-art OTS

After finding that the *Mbur*PylRS OTS was the best PylRS even in multi-site ncAA incorporation we compared it with one of the most efficient OTS described in the literature, for *E*. *coli, Mj*TyRS (Figure 11).

The *Mj*TyrRS system, along with the TyrRS system from *Archaeoglobus fulgidus* (*Af*TyrRS), is one of the well-known OTS that can produce target proteins with in-frame stop codons at wild-type level.²⁻⁴ For comparison two *Mbur*PylRS and two *Mj*TyrRS variants were used. The MjONB-Dopa OTS was selected because its high efficiency is well documented in our group.^{3,5} The second variant was chosen according to efficiency considerations.

Methods: The *Mj*TyrRS variants are in a different plasmid system (pUltra) than the PylRS OTS (pTECH). To estimate the differences in efficiency despite different OTS setups, the fluorescence signals were normalized to the corresponding wild-type target protein signal (sfGFP without an in-frame stop codon). All selected genes were assembled into plasmids by Golden Gate cloning and confirmed by DNA sequencing. Afterwards the ncAA incorporation efficiency was determined by intact cell fluorescence measurements, *E*. *coli* cells were used. Electrocompetent cells were transformed with the orthogonal translation system and reporter plasmids. LB agar plates for plating contained 1 % glucose and corresponding antibiotics. Single colonies of clones were used for inoculation of 2 mL LB (in 14 mL tubes) with 1 % glucose and appropriate antibiotics and grown to saturation overnight. Assays were conducted in 96-well plate format. Cultures were added to each well at 1:100 dilution in ZYP-5052 auto induction medium to a final volume of 100 µL supplemented with antibiotics and ncAAs. Cells were grown in black µ-plates (Greiner Bio-One, Kremsmünster, Austria) covered with a gas permeable foil (Breathe-Easy^{®}, (Diversified Biotech, Doylestown, PA)) with orbital shaking for 24 h at 37 °C. For endpoint measurements (Tecan M200), the plate foil was removed, and fluorescence measured with an 85 gain setting. For OD₆₀₀ measurements, 50 µL of ZYP-5052 medium was introduced into clear 96-well µ-plates and 50 µL of culture added. Excitation and emission wavelengths for fluorescence measurements were set to 481 nm and 511 nm, respectively. Fluorescence values were normalized to the corresponding OD₆₀₀. Biological triplicates were used for measurements of each aaRS construct. Relative fluorescence was normalized to the highest value. The data (incl. standard deviation) represents the mean of three biological replicates.

In general, the efficiency of suppressing a single in-frame stop codon is comparable. Surprisingly, the MburPylRS-based OTS suppressed multiple in-frame stop codons with far higher efficiency than the *Mj*TyrRS OTS seen by the fact that at 1 mM ncAA concentration, the decrease in efficiency suppressing multiple stop codons is much lower. The efficiency of *Mbur*PylRS suppressing five in-frame stop codons is 2.5 to 3 times higher than of *Mj*TyrRS. The high performance of *Mbur*PylRS in a RF1 deficient strain may indicate superior performance when used in conjunction with strains that have liberated codons.

### Example 4: Recombinant protein production - Large scale

For selected ncAA OTS combinations shake flask cultivations (15 mL culture) for protein production was performed to show that the small scale recombinant protein expressions results are also valid for larger scale production

For expression of the SUMO-sfGFP variants, E. *coli* strains were used in 15 mL ZYP-5052 medium supplemented with 10 mM ncAA and appropriate antibiotics. The expression medium was inoculated with a starter culture (1:100). Shake flasks were incubated for 24 h at 37 °C while shaking at 200 rpm. Cells were harvested by centrifugation and stored at -80 °C or directly used for protein purification. Harvested cell pellets were resuspended (50 mM sodium phosphate, 300 mM NaCl, 20 mM imidazole, pH 8.0) and lysed with B PER^{®} Bacterial Protein Extraction Reagent (ThermoFisher Scientific, Waltham, MA, USA) according to their protocol, with addition of phenylmethanesulfonyl fluoride (PMSF, 1 mM final concentration), DNAse and RNAse. Cleared lysates were loaded onto a equilibrated Ni-NTA column and purified via a peristaltic pump (Pharmacia Biotech, Stockholm, SE). After washing with 10 column volumes of resuspension buffer, elution buffer (50 mM sodium phosphate, 300 mM NaCl, 500 mM imidazole, pH 8.0) was applied to elute the his-tagged target proteins. The first 2 mL (covering the dead volume) were discarded. Afterwards, the eluate (1 mL) was collected and dialyzed in cellulose film tubings against 1 L buffer (50 mM sodium phosphate, 300 mM NaCl, pH 8.0) for at least 2 h with three buffer changes. Concentrations of purified reporter proteins were determined by measuring the sfGFP chromophore absorption at 488 nm or absorption at 280 nm. Correct ncAA incorporation was verified by ESI-MS (Waters Xevo G2-XS QTof). A Waters Acquity UPLC protein BEH C4 column (300 Å, 1.7 µm, 2.1 mm × 50 mm) was used and gradient at a flow rate of 0.3 mL/min: A: 0.01% formic acid in H2O; B: 0.01% formic acid in MeCN. 5-95% B 0-6 min). Mass analysis was conducted with a Waters Xevo G2-XS QTof analyzer (positive mode, cone voltage=40 kV). Deconvoluted raw data were analyzed employing the maximum entropy deconvolution algorithm and plotted with QtiPlot (Figure 15).

In general, all larger scale protein productions results are in agreement with the small scale fluorescent assays, confirming the higher ncAA incorporation efficiency of *Mbur*PylRS (Figure 13).

### Example 5: Generation of the modified tRNA and comparison

It was found that a tRNA from *M. alaskense* had a similar sequence to that of *M. burtonii* with one mutation. The mutation was predicted to have large consequences in terms of thermodynamic stability. Therefore, it was tested if the performance of the *Mbur*PylRS could be increased by using the *M. alaskense* tRNA.

For this, the two selected tRNA genes were assembled into plasmids by Golden Gate cloning and confirmed by DNA sequencing. Afterwards the ncAA incorporation efficiency was determined by intact cell fluorescence measurements, *E*. *coli* cells were used. Electrocompetent cells were transformed with the orthogonal translation system and reporter plasmids. LB agar plates for plating contained 1 % glucose and corresponding antibiotics. Single colonies of clones were used for inoculation of 2 mL LB (in 14 mL tubes) with 1 % glucose and appropriate antibiotics and grown to saturation overnight. Assays were conducted in 96-well plate format. Cultures were added to each well at 1:100 dilution in ZYP-5052 auto induction medium to a final volume of 100 µL supplemented with antibiotics and ncAAs. Cells were grown in black µ-plates (Greiner Bio-One, Kremsmünster, Austria) covered with a gas permeable foil (Breathe-Easy^{®}, (Diversified Biotech, Doylestown, PA)) with orbital shaking for 24 h at 37 °C. For endpoint measurements (Tecan M200), the plate foil was removed, and fluorescence measured with an 85 gain setting. For OD₆₀₀ measurements, 50 µL of ZYP-5052 medium was introduced into clear 96-well µ-plates and 50 µL of culture added. Excitation and emission wavelengths for fluorescence measurements were set to 481 nm and 511 nm, respectively. Fluorescence values were normalized to the corresponding OD₆₀₀. Biological triplicates were used for measurements of each aaRS construct. Relative fluorescence was normalized to the highest value. The data (incl. standard deviation) represents the mean of three biological replicates

Remarkably, the ncAA incorporation efficiency was increased when using the tRNA of *M. alaskense* by up to 50% depending on the substrate.

### Example 6: Stochastic incorporation of Sac and pLeu into sfGFP and amilCP

For the stochastic incorporation of Sac and pLeu into sfGFP and amilCP (chromo protein of Acropora millepora) the anticodon of the tRNA(Mbur) on the pTECH plasmid was changed to target the desired sense codon. Correct change was confirmed by sanger sequencing. Afterwards, *E*. *coli* strains were used in 15 mL ZYP-5052 medium supplemented with 1 mM ncAA and appropriate antibiotics. The expression medium was inoculated with a starter culture (1:100). Shake flasks were incubated for 24 h at 37 °C while shaking at 200 rpm. Cells were harvested by centrifugation and stored at -80 °C or directly used for protein purification. Harvested cell pellets were resuspended (50 mM sodium phosphate, 300 mM NaCl, 20 mM imidazole, pH 8.0) and lysed with B PER^{®} Bacterial Protein Extraction Reagent (ThermoFisher Scientific, Waltham, MA, USA) according to their protocol, with addition of phenylmethanesulfonyl fluoride (PMSF, 1 mM final concentration), DNAse and RNAse. Cleared lysates were loaded onto a equilibrated Ni-NTA column and purified via a peristaltic pump (Pharmacia Biotech, Stockholm, SE). Afterwashingwith 10 column volumes of resuspension buffer, elution buffer (50 mM sodium phosphate, 300 mM NaCl, 500 mM imidazole, pH 8.0) was applied to elute the his-tagged target proteins. The first 2 mL (covering the dead volume) were discarded. Afterwards, the eluate (1 mL) was collected and dialyzed in cellulose film tubings against 1 L buffer (50 mM sodium phosphate, 300 mM NaCl, pH 8.0) for at least 2 h with three buffer changes. Concentrations of purified reporter proteins were determined by measuring the sfGFP absorption at 488 nm, the amilCP absorption at 588 nm or absorption at 280 nm. Correct ncAA incorporation was verified by ESI-MS (Waters Xevo G2-XS QTof). A Waters Acquity UPLC protein BEH C4 column (300 Å, 1.7 µm, 2.1 mm × 50 mm) was used and gradient at a flow rate of 0.3 mL/min: A: 0.01% formic acid in H2O; B: 0.01% formic acid in MeCN. 5-95% B 0-6 min). Mass analysis was conducted with a Waters Xevo G2-XS QTof analyzer (positive mode, cone voltage=40 kV). Deconvoluted raw data were analyzed employing the maximum entropy deconvolution algorithm and plotted with QtiPlot (Figure 21-23).

### Example 7: Proof of functionality in mammalian systems in vivo

HEK293T cells were maintained at 37 °C in a humidified 5% CO₂ atmosphere in Dulbecco's modified Eagle's Medium (DMEM, Corning), supplemented with 10% (volume/volume) fetal bovine serum (Sigma Aldrich) and 2 mM L-Glutamine (Corning). The ncAAs (0.45 mM Sac or 0.5 mM pLeu final concentration) were added together with fresh growth medium 1 h prior to transfection. For the fluorescence readout in clear 96-well plates, 4 × 10⁵ HEK293T cells were seeded per well (with spaces between the replicates on the plate) in 100 µl DMEM supplied with 0.2 mM Leu and transfected the day after with 100 ng total DNA of the reporter plasmid (containing EGFP(Y183 amber and mRFP)) and the plasmid containing the OTS in a 1:1 ratio (50 ng reporter and 50 ng OTS), using the Lipofectamine 2000 (ThermoFisher Scientific) protocol. 48 h later, total green and red fluorescence of whole cells were quantified using a plate reader (FLUOstar Omega)(Figure 19 and 20). Suppression efficiency is stated as the percentage of wild type fluorescence of EGFP containing no in-frame stop codon. The values were obtained by using the EGFP/mRFP ratio. To further improved the mRS performance in HEK293T cells the construct is also tested with a nuclear export sequence (SEQ ID No: 11) which increase the incorporation efficiency (Figure 19 and 20).

### References:

Berg, J. M.; Tymoczko, J. L.; Gatto, G. J. jr.; Stryer, L. Biochemistry, 8th ed.; W. H. Freeman and Company: New York, 2015.

Saier, M. H. Understanding the Genetic Code. J. Bacteriol. 2019, 201 (15), 1-12. https://doi.org/10.1128/JB.00091-19.

## Claims

1. Pyrrolysyl-tRNA synthetase mutants (mRS) for the incorporation of amino acids with non-proteinogenic functional groups (ncAAs) into recombinant proteins by targeted and site-specific tRNA elongation, deriving from *Methanococcoides burtonii* (*Mbur*PylRS), having at least 90% sequence identity with *Mbur*PylRS and comprising a modification in the catalytic center **characterized in** the amino acid exchange Y346F.

2. The mRS according to claim 1 described by SEQ ID No.: 1 and comprising one or more amino acid modifications selected from the group consisting of N308Q, N308L, N308M, N308G, N308A, C310A, C310G, C310W, S379T and W379T.

3. The mRS according to any of the previous claims generated from or based on a humanized DNA coding sequence.

4. The mRS according to any of the previous claims additionally incorporating a nuclear export signal.

5. The mRS according to any of the claims 1 to 4 integrating ncAAs into a newly synthesized polypeptide or protein by elongating the growing amino acid sequence with tRNAs selected from the group of homologous tRNAs and heterologous tRNAs.

6. The mRS according to any of the claims 1 to 5 integrating ncAAs into a newly synthesized polypeptide or protein by elongating the growing amino acid sequence with modified tRNAs **characterized by** the SEQ ID No: 2.

7. The mRS according to any of the claims 1 to 6, wherein the integration of ncAAs into the newly synthesized polypeptide or protein is directed to a nucleotide triplet in the mRNA selected from the group of termination codons and alternative termination codons comprising TAG, TAA, TGA, AGA, AGG, TCA and TTA.

8. The mRS according to any of the claims 1 to 6, wherein the integration of ncAAs into the newly synthesized polypeptide or protein is directed to a nucleotide triplet in the mRNA selected from the group of sense codons and modified sense codons comprising TTG (leucin), CTG (leucin), TCC (serine) and ATA (isoleucine) and all other sense codons.

9. Modified tRNA consisting of SEQ ID No: 2 suitable for elongation by the mRS according to claim 6.

10. Vector comprising a nucleic acid sequence encoding the mRS according to claim 1-8 and optionally, comprising the nucleic acid sequence encoding one or more tRNAs selected from homologous, heterologous and modified tRNAs according to claim 9.

11. Vector according to claim 10 selected from the group of pro- and eukaryotic expression plasmids, linear pro- and eukaryotic expression systems, viral vectors and viral expression systems.

12. Method for the incorporation of amino acids with non-proteinogenic functional groups (ncAAs) into recombinant proteins comprising the steps of:
- selecting, pre-modifying and providing in a prokaryotic or eukaryotic host cell the nucleic acid (preferably a mRNA) to be targeted having a triplet suitable for site specific elongation,
- providing a vector encoding and expressing the mRS according to any of the claims 1-8,
- providing the same or an additional vector encoding and expressing one or more tRNA selected from homologue tRNAs, heterology tRNAs or modified tRNAs according to claim 9 and mixtures thereof;
- transfecting, infecting and incubating a host cell culture with one or more of the preselected vectors under suitable cell culture conditions,
- isolating the synthesised recombinant protein from the host cells, the supernatant and/or the host cell culture.

13. Kit comprising one or several containers comprising
- one or more vectors for the expression of the mRS according to any of the claims 1-8 in suitable buffers;
- one or more vectors for the expression of the nucleic acid sequence encoding one or more tRNAs selected from heterologous, homologous and/or modified tRNAs according to claim 9 in suitable buffers;
- optionally comprising a cloning plasmid for introducing the mRNA of choice to the host cell system;
- optionally, comprising suitable host cells and suitable cell culture propagation media;
- instructions how to use the kit.

14. Use of the mRS according to any of the claims 1-8, use of the kit according to claim 14 for the generation and/or production of recombinant polypeptides and/or protein incorporating in pre-determined and site-specific locations amino acids with non-proteinogenic functional groups for further coupling or conjunction in protein-drug and/or protein-protein conjugants.

15. Protein production platform for the generation, modification and production of recombinant protein employing
- a host cell culture carrying and/or expressing a target sequence for site-specific manipulation of polypeptide or protein to be synthesised;
- one or more vectors for the expression of the mRS according to any of the claims 1-8;
- one or more vectors for the expression of the nucleic acid sequence encoding one or more tRNAs selected from heterologous or modified tRNAs according to claim 9.
